# EUROPÄISCHE PATENTSCHRIFT

(11) **EP 0 381 661 B1**
(45) Veröffentlichungstag und Bekanntmachung des Hinweises auf die Patenterteilung: **24.05.1995**
(21) Anmeldenummer: 90890015.2
(22) Anmeldetag: 23.01.1990
(51) Int. Cl.: C07D 333/32, A61K 31/38

(54) **1-E3-(2-Hydroxy-3-alkylaminopropoxy)-2-thienyl]-3-phenyl-1-propanone und Verfahren zu deren Herstellung**
1-E3-(2-hydroxy-3-alkylaminopropoxy)-2-thienyl]-3-phenyl-1-propanon and process for its preparation
1-E3-(2-hydroxy-3-alkylaminopropoxy)-2-thiényl]-3-phényl-1-propanone et procédé pour sa préparation

(30) Priorität: 24.01.1989 AT 130/89
(43) Veröffentlichungstag der Anmeldung: 08.08.1990
(73) Patentinhaber: Laevosan-Gesellschaft m.b.H., A-4020 Linz (AT)
(72) Erfinder: Binder, Dieter, Prof. Dr., A-1190 Wien (AT); Greier, Gerhard, Dr. Dipl.-Ing., A-4020 Linz (AT)
(74) Vertreter: Pawloy, Heinrich, Dr.

(56) Entgegenhaltungen:
- EP-A- 0 053 603
- EP-A- 0 233 173
- EP-A- 0 266 336
- DE-A- 3 316 155

## Beschreibung

Die Erfindung betrifft Medikamentvorläufer, d.h. neue therapeutisch wertvolle Derivate des 1-[3-(2-Hydroxy-3-alkylaminoproproxy)-2-thienyl]-3-phenyl-1-propanons der allgemeinen Formel (I) 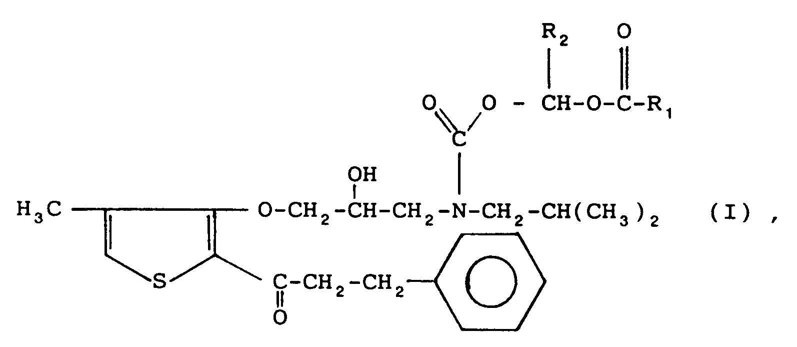
worin R₁ gesättigtes oder ungesättigtes Alkyl, Aryl, Heteroaryl wie Thienyl, Furyl oder Pyridyl, Alkylaryl, Alkylheteroaryl, Alkoxy, Alkoxyaryl oder Alkoxyheteroaryl mit jeweils 1 bis 5 C-Atomen in der Alkylgruppe bedeutet und R₂ nieders Alkyl ist, und ein Verfahren zu deren Herstellung.

Thienylderivate der Formel (II) 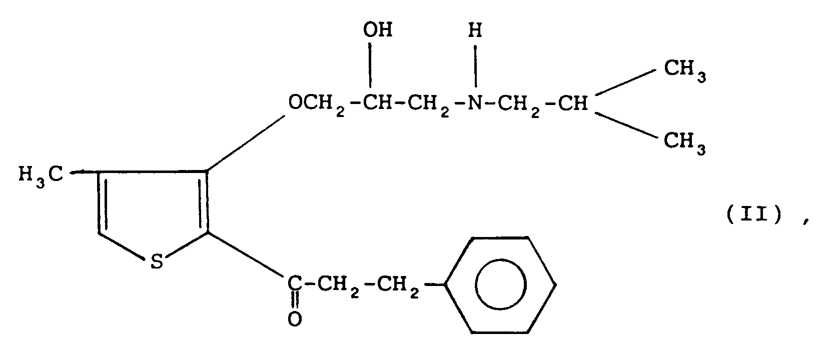
welche am Stickstoffatom unsubstituiert sind, sind bereits bekannt. Diese Verbindungen besitzen schon in niedrigen Dosen hervorragende antiarrhythmische Aktivität und sind auch oral wirksam. Bei der pharmazeutischen Anwendung dieser bekannten Verbindungen stellte sich jedoch heraus, daß sie vom menschlichen oder tierischen Körper nur schlecht resobiert werden können. Es wurden daher Versuche unternommen, die bekannten, bei Herzrhythmusstörungen hervorragend wirksamen Substanzen in eine Form überzuführen, welche vom menschlichen oder tierischen Körper leicht resorbierbar ist und trotzdem die gewünschte pharmazeutische Wirksamkeit nicht verliert.

Überraschenderweise wurde nun gefunden, daß man pharmazeutisch praktisch gleich wirksame Verbindungen erhält, wenn die Aminogruppe der bekannten Verbindungen nach der in Formel (I) angegebenen Weise substituiert wird.

Es wird angenommen, daß die Verbindungen der Formel (I) nach ihrer Resorption in die Verbindungen der Formel (II) übergeführt werden.

Die neuen Verbindungen besitzen somit nicht nur eine ausgezeichnete Wirkung gegen Herzrhythmusstörungen, sondern sind darüberhinaus außerdem vom menschlichen oder tierischen Körper wesentlich besser resorbierbar als die bisher bekannten Verbindungen.

Die Erfindung bezieht sich weiterhin auf ein Verfahren zur Herstellung der Verbindungen der allgemeinen Formel (I), welches darin besteht, daß eine Verbindung der Formel (II) 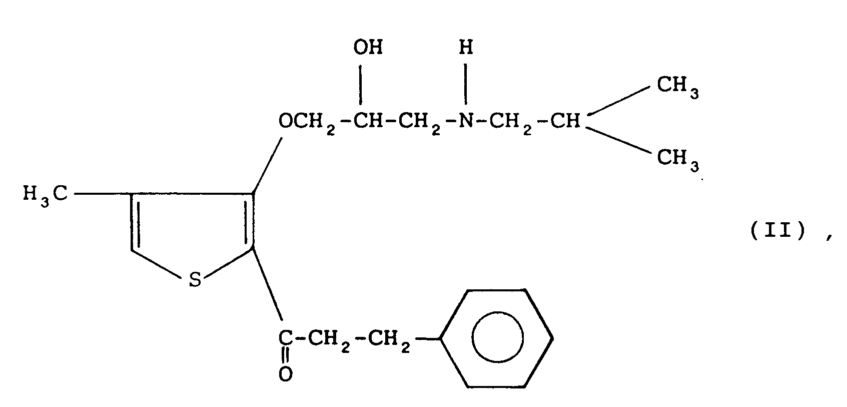
mit einer Verbindung der allgemeinen Formel (III) 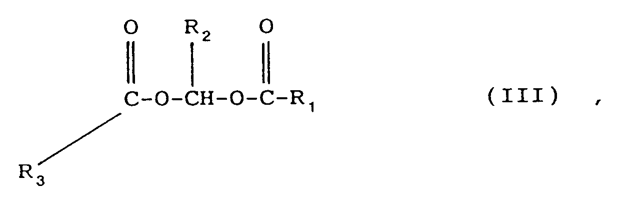
worin R₁ und R₂ die oben angegebenen Bedeutungen haben und R₃ ein durch eine Aminogruppe substituierbarer Rest, wie z.B. p-Nitrophenoxy, ist, umgesetzt wird. Vorzugsweise wird als Verbindung der Formel (III) α-Acetoxyäthyl-p-nitrophenylcarbonat eingesetzt und die Reaktion in Hexamethylphosphorsäuretriamid bei Raumtemperatur durchgeführt.

Das folgende Beispiel soll das erfindungsgemäße Verfahren zur Herstellung der neuen Verbindungen erläutern, ohne die Erfindung jedoch darauf einzuschränken.
**Beispiel:** Eine Lösung von von 9,8 g (26,09 mMol) 1-[3-(2-Hydroxy-3-(2-methylpropylamino)-propoxy)-4-methyl-2-thienyl]-3-phenyl-1-propanon und 7 g (26,00 mMol) α-Acetoxyäthyl-p-nitrophenylcarbonat in 85 ml trockenem Hexamethylphosphorsäuretriamid wird 2 h bei Raumtemperatur gerührt.

Das Reaktionsgemisch wird auf 300 ml Wasser gegossen und mit Äther extrahiert. Die organische Phase wird mit 1N Natronlauge und Wasser gewaschen, über Natriumsulfat getrocknet und eingedampft. Es werden 12,8 g öliger Rückstand erhalten, welcher säulenchromatographisch gereinigt wird.
Säulenchromatographie: Kieselgel,
LM für 1. Säule: Dichlormethan/Äthanol = 12/1
LM für 2. Säule: Toluol/Äthanol = 12/1
DC: LM Dichlormethan/Äthanol = 12/1; Rf = 0,65
Ausbeute: 11,6 g blaßgelbes zähes Öl (87,9 %)
1H-NMR (DMSO) δ : 7,22 (s, 5H, Bz-H), 7,10 (s, 1H, Th-H), 6,84 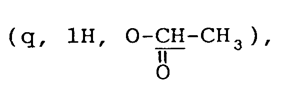
4,62 (m, 1H, CH₂-CH-OH), 4,06 (m, 3H, O-CH₂, -OH), 3,56-2,87 (m, 8H, 2xN-CH₂+COCH₂-Bz), 2,16 (s, 3H, Th-CH₃), 2,01 (s, 3H, CO-CH₃), 1,86 (m, 1H, -CH-(CH₃)₂), 1,45 (d, 3H, -OCH-CH₃), 0,89 (d, 6H, -CH-(CH₃)₂).

## Patentansprüche

1. Derivate des 1-[3-(2-Hydroxy-3-alkylaminoproproxy)-2-thienyl]-3-phenyl-1-propanons der allgemeinen Formel (I) 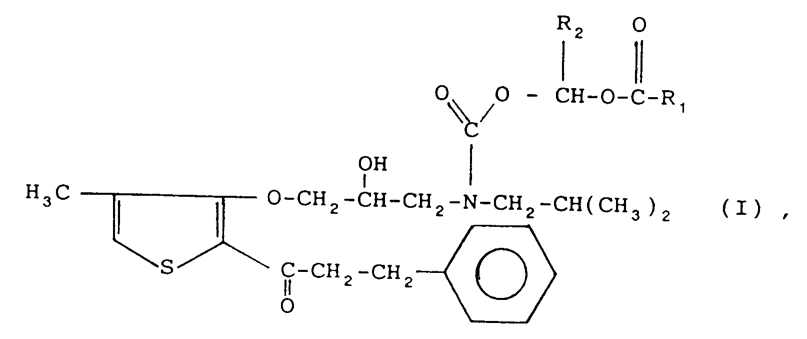 worin R₁ gesättigtes oder ungesättigtes Alkyl, Aryl, Heteroaryl wie Thienyl, Furyl oder Pyridyl, Alkylaryl, Alkylheteroaryl, Alkoxy, Alkoxyaryl oder Alkoxyheteroaryl mit jeweils 1 bis 5 C-Atomen in der Alkylgruppe bedeutet und R₂ niederes Alkyl ist.

2. 1-[3-[2-Hydroxy-3-[N-[(1-acetyloxyäthoxy)carbonyl]-N-(2-methylpropylamino)]propoxy]-4-methyl-2-thienyl]-3-phenyl-1-propanon.

3. Verfahren zur Herstellung der Verbindungen der Formel (I), wie in Anspruch 1 definiert, dadurch gekennzeichnet, daß eine Verbindung der Formel (II) 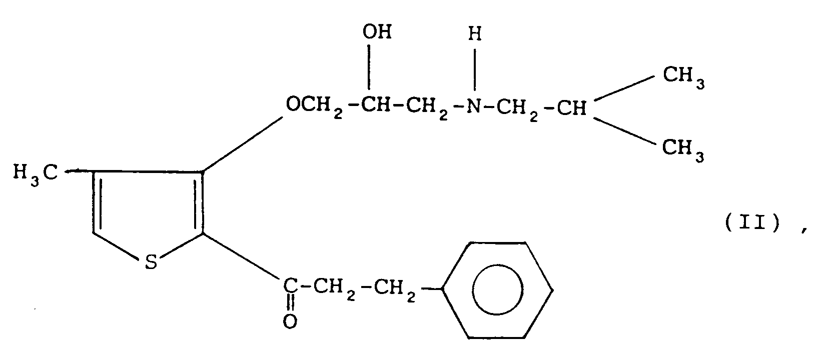 mit einer Verbindung der allgemeinen Formel (III) 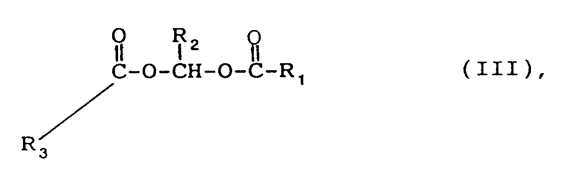 worin R₁ und R₂ die oben angegebenen Bedeutungen haben und R₃ ein durch eine Aminogruppe substituierbarer Rest, wie z.B. p-Nitrophenoxy, ist, umgesetzt wird.

## Claims

1. Derivatives of 1-[3-(2-hydroxy-3-alkylaminopropoxy)-2-thienyl]-3-phenyl-1-propanone of the general formula (I) 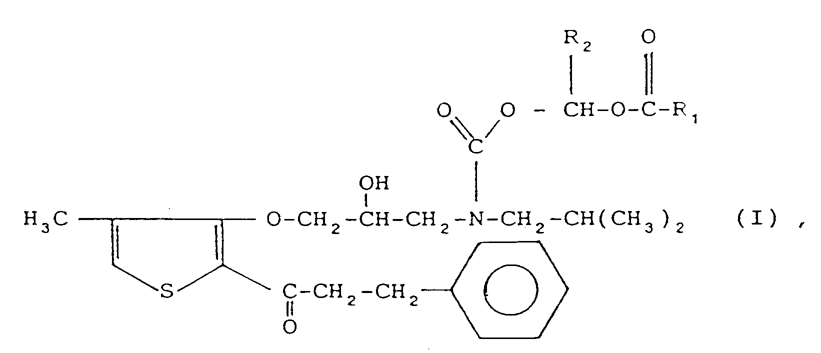 wherein R₁ represents saturated or unsaturated alkyl, aryl, heteroaryl, such as thienyl, furyl or pyridyl, alkyl aryl, alkyl heteroaryl, alkoxy, alkoxy aryl or alkoxy heteroaryl, each having 1 to 5 C-atoms in the alkyl group and R₂ is lower alkyl.

2. 1-[3-[2-Hydroxy-3-[N-[(1-acetyloxyethoxy)carbonylalkyl]-N-(2-methylpropylamino)]propoxy]-4-methyl-2-thienyl]-3-phenyl-1-propanone.

3. A process for preparing the compounds of formula (I) as defined in claim 1, characterized in that a compound of the formula (II) 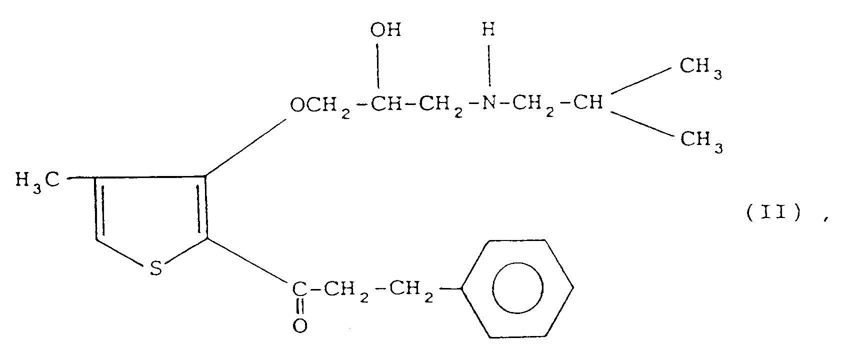 is reacted with a compound of the general formula (III) 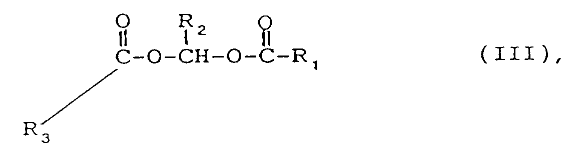 wherein R₁ and R₂ are as defined above and R₃ is a residue substituted by an amino group, such as, e.g., p-nitrophenoxy.

## Revendications

1. Dérivés de 1-[3-(2-hydroxy-3-alcoylaminopropoxy)-2-thiényl]-3-phényl-1-propanone de formule générale (I) 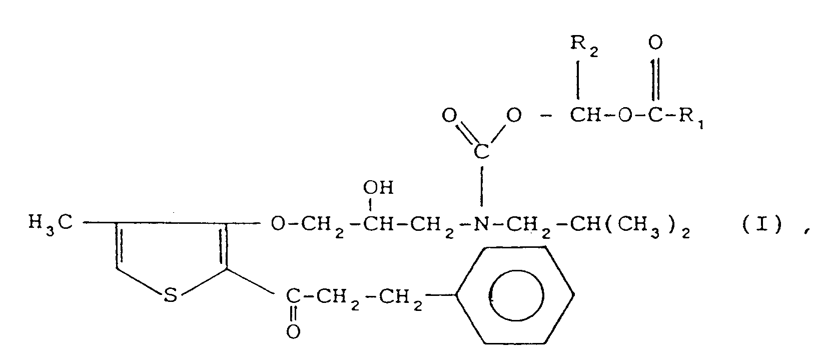 dans laquelle R₁ représente alcoyle saturé ou insaturé, aryle, hétéroaryle, tel que thiényle, furyle ou pyridyle, alcoyle aryle, alcoyle, hétéroaryle, alcoxy, alcoxy aryle ou alcoxy hétéroaryle, ayant chacun 1 à 5 atomes de carbone dans le groupe alcoyle et R₂ est alcoyle inférieur.

2. 1-[3-[2-Hydroxy-3-[N-[(1-acétyloxyéthoxy)carbonyl]-N-(2-méthylpropylamino)]propoxy]-4-méthyl-2-thiényl]-3-phényl-1-propanone.

3. Procédé pour la préparation des composés de formule (I) telle que définie dans la revendication 1, caractérisé en ce que l'on fait réagir un composé de formule (II) 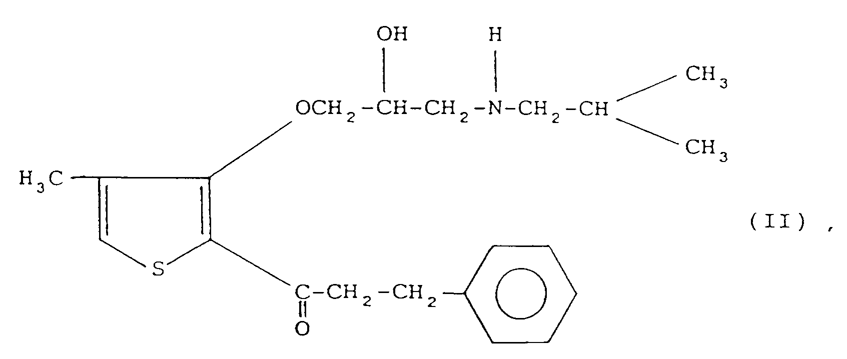 avec un composé de formule générale (III) 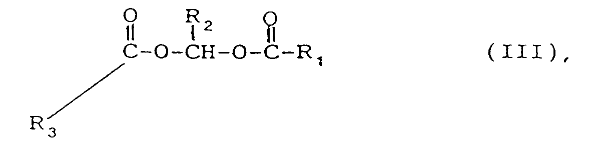 dans laquelle R₁ et R₂ sont tels que définis ci-dessus et R₃ est un résidu substitué d'un groupe amino, comme par exemple p-nitrophénoxy.
